# EUROPÄISCHE PATENTANMELDUNG

(11) **EP 0 959 091 A1**
(43) Veröffentlichungstag der Anmeldung: **24.11.1999**
(21) Anmeldenummer: 99109050.7
(22) Anmeldetag: 07.05.1999
(51) Int. Cl.: C08G 73/10, C08G 73/14, C08G 73/02, A61K 7/48, C11D 3/37, A61K 7/06

(54) **Copolymere hydrophob modifizierte Polyasparaginsäureesteramide und ihre Verwendung**

(30) Priorität: 20.05.1998 DE 19822599
(71) Anmelder: Th. Goldschmidt AG, 45127 Essen (DE)
(72) Erfinder: Grüning, Burghard Dr., 45134 Essen (DE); Simpelkamp, Jörg Dr., 45130 Essen (DE); Weitemeyer, Christian Dr., 45134 Essen (DE)

(57) **Zusammenfassung**

Diese Erfindung betrifft copolymere Polyasparaginsäureesteramide, welche mit Alkyl- oder Alkenylresten mit 6-30 C-Atomen modifiziert sind, ihre Herstellung sowie ihre Verwendung.

Von Polyaminosäuren abgeleitete Copolymere, die zu mindestens 75 Mol.-% der vorhandenen Einheiten aus Struktureinheiten der allgemeinen Formeln (I),(II) oder (III) 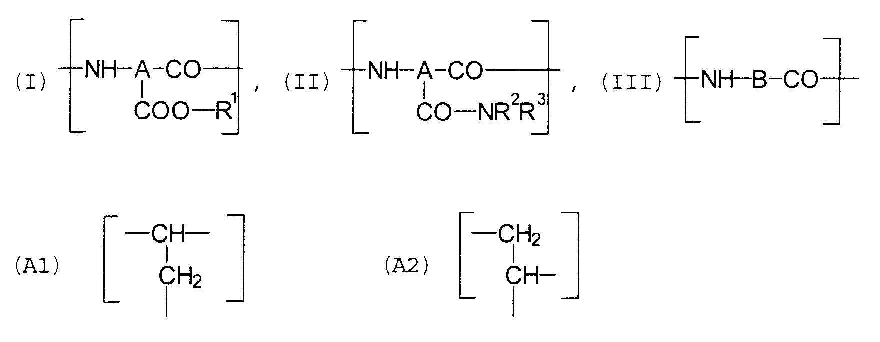 bestehen, in denen die Strukturelemente A gleiche oder verschiedene trifunktionelle Kohlenwasserstoffradikale mit 2 C-Atomen des Typs (A1) oder (A2) sind, worin
- R¹: die Bedeutung von R⁴, R⁵, und R⁶ haben kann,
- R²: für gleiche oder verschiedene, geradkettige oder verzweigte, gesättigte oder ungesättigte Alkyl-, Alkenyl- oder Arylreste mit 1 bis 30 C-Atomen für Hydroxy- oder Aminoalkylreste mit 1 bis 22 C-Atomen und 1 bis 6 Hydroxy- und/oder Aminogruppen und/oder deren Acylierungsprodukte mit 1 bis 22 C-Carbonsäuren oder die Bedeutung von R⁵ annimmt, und
- R³: gleich Wasserstoff oder R² ist,
- R⁴: für ein oder mehrere Reste aus der Gruppe der Alkali-, Erdalkalimetalle, Wasserstoff oder Ammonium, [NR⁷R⁸R⁹R¹⁰]⁺ , worin
R⁷ bis R¹⁰ unabhängig voneinander Wasserstoff, Alkyl oder Alkenyl mit 1 bis 22 C-Atomen oder Hydroxyalkyl mit 1 bis 22 C-Atomen ist, mit 1 bis 6 Hydroxygruppen.
- R⁵: für gleiche oder verschiedene, geradkettige oder verzweigte, gesättigte oder ungesättigte Alkyl- oder Alkenylreste R¹¹ mit 6 bis 30 C-Atomen oder Radikale der Struktur -Y-R¹¹, wobei Y eine Oligo- oder Polyoxyalkylenkette mit 1 bis 100 Oxyalkyleneinheiten ist, und
- R⁶: für gleiche oder verschiedene, geradkettige oder verzweigte, gesättigte oder ungesättigte Alkyl- oder Alkenylreste mit 1 bis 5 C-Atomen steht,
und wenigstens jeweils eine Einheit der allgemeinen Formel (I), bei dem der Rest R¹ die Bedeutung von R⁴ annimmt, und wenigstens eine Einheit der allgemeinen Formel (II) enthalten ist, und die Einheiten der allgemeinen Formel (III) proteinogene oder nicht proteinogene Aminosäuren sind und zu nicht mehr als 20 Gew.-%, bezogen auf copolymere Polyasparaginsäurederivate, enthalten sind.

## Beschreibung

Diese Erfindung betrifft copolymere Polyasparaginsäureesteramide, welche mit Alkyl- oder Alkenylresten mit 6-30 C-Atomen modifiziert sind, ihre Herstellung sowie ihre Verwendung.

Polyaminosäurederivate, insbesondere Polyasparaginsäure, haben in jüngster Zeit aufgrund ihrer Eigenschaften, insbesondere ihrer biologischen Abbaubarkeit und Naturnähe, besondere Aufmerksamkeit gefunden. Es werden u.a. Anwendungen als biologisch abbaubare Komplexierungsmittel, Enthärter und Waschmittel-Builder vorgeschlagen. Polyasparaginsäure wird i.A. durch alkalische Hydrolyse der unmittelbaren Synthesevorstufe Polysuccinimid (PSI, Anhydropolyasparaginsäure), dem cyclischen Imid der Polyasparaginsäure gewonnen. PSI kann beispielsweise nach EP 0 578 449 A, WO 92/14753, EP 0 659 875 A oder DE 44 20 642 A aus Asparaginsäure hergestellt werden oder ist beispielsweise nach DE 36 26 672 A, EP 0 612 784 A, DE 43 00 020 A oder US 5 219 952 A aus Maleinsäurederivaten und Ammoniak zugänglich. Für diese üblichen Polyasparaginsäuren werden u.a. Anwendungen als Inkrustationsinhibitor, Builder in Waschmitteln, Düngemitteladditiv und Hilfsstoff in der Gerberei vorgeschlagen.

Die von verschiedenen Arbeitsgruppen beschriebene Umsetzung von Polysuccinimid mit Aminen führt zu Polyasparaginsäureamiden (Kovacs et al., J. Med. Chem. 1967, 10, 904-7; Neuse, Angew. Makromol. Chem. 1991, 192, 35-50). Die Ringöffnung von Polysuccinimid mit Polyaminen und die nachfolgende alkalische Hydrolyse zur Herstellung von Polyasparaginsäurederivaten für Anwendungen als Superabsorber wird beispielsweise in der WO 95/35337 beschrieben.
US 5,292,858 A beschreibt die Umsetzung von Maleinsäuremonoestern mit Ammoniak oder Ammoniak und Aminen zu Polysuccinimid oder amidgruppen-enthaltenden Polysuccinimidderivaten, welche im Endprodukt keine Estergruppen mehr enthalten.

Von besonderem Interesse u.a. für Anwendungen als Emulgator, Dispergiermittel und Tensid sind copolymere Polyasparaginsäureester, welche partiell mit langkettigen Fettalkoholen oder deren Derivaten verestert sind. Derartige Verbindungen sind auf Basis von Maleinsäuremonoestern und Ammoniak leicht zugänglich, wie aus der DE 195 45 678 A beziehungsweise der EP 96 118 806.7 A hervorgeht.

In ihren anwendungstechnischen Eigenschaften weisen diese Derivate jedoch verschiedene Nachteile auf, insbesondere bezüglich der Temperatur- und Langzeitstabilität der Zubereitungen beispielsweise im Bereich kosmetischer W/O und O/W-Emulsionen.

Aufgabe der Erfindung war daher die Bereitstellung copolymerer Polyasparaginsäureesterderivate, welche verbesserte Anwendungseigenschaften besitzen.

Die Aufgabe wird erfindungsgemäß gelöst durch copolymere Polyasparaginsäureester mit Ester- und Amidgruppen in der Polymerseitenkette, welche aus Derivaten α,β-ungesättigter Carbonsäuren und Ammoniak hergestellt werden. Dabei bewirkt das gleichzeitige Vorhandensein von Ester- und Amidgruppen im Molekül überraschenderweise besonders vorteilhafte Anwendungseigenschaften.

Die eingesetzten, von Polyasparaginsäure abgeleiteten Copolymeren bestehen zu wenigstens 75 Mol.-% der vorhandenen Einheiten aus Struktureinheiten der allgemeinen Formeln (I), (II) und (III), wobei die Strukturelemente A gleiche oder verschiedene trifunktionelle Kohlenwasserstoffradikale mit 2 C-Atomen der Struktur (A1) oder (A2) sind, 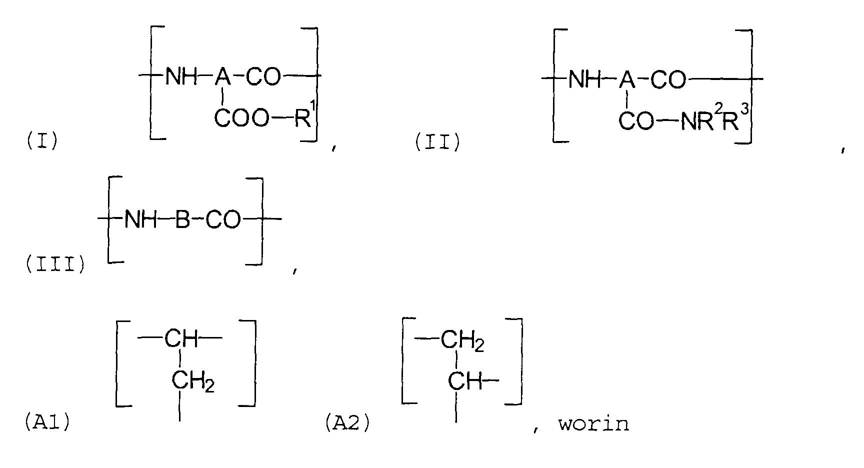
- R¹: die Bedeutung von R⁴, R⁵, und R⁶ hat,
- R²: für gleiche oder verschiedene, geradkettige oder verzweigte, gesättigte oder ungesättigte Alkyl-, Alkenyl- oder Arylreste mit 1 bis 30 C-Atomen, für Hydroxy- oder Aminoalkylreste mit 1 bis 22 C-Atomen und 1 bis 6 Hydroxy- und/oder Aminogruppen und/oder deren Acylierungsprodukte mit 1 bis 22 C-Carbonsäuren oder die Bedeutung von R⁵ annimt, und
- R³: gleich Wasserstoff oder R² ist,
- R⁴: für ein oder mehrere Reste aus der Gruppe der Alkali-, Erdalkalimetalle, Wasserstoff oder Ammonium, [NR⁷R⁸R⁹R¹⁰]⁺, worin R⁷ bis R¹⁰ unabhängig voneinander Wasserstoff, Alkyl oder Alkenyl mit 1 bis 22 C-Atomen oder Hydroxyalkyl mit 1 bis 22 C-Atomen und 1 bis 6 Hydroxygruppen ist,
- R⁵: für gleiche oder verschiedene, geradkettige oder verzweigte, gesättigte oder ungesättigte Alkyl- oder Alkenylreste R¹¹ mit 5 bis 30 C-Atomen oder Radikale der Struktur -Y-R¹¹ wobei Y eine Oligo- oder Polyoxyalkylenkette mit 1 bis 100 Oxyalkyleneineiten ist, und
- R⁶: für gleiche oder verschiedene, geradkettige oder verzweigte, gesättigte oder ungesättigte Alkylreste mit 1 bis 5 C-Atomen steht, und wenigstens jeweils ein Baustein (I), bei dem der Rest R¹ die Bedeutung von R⁴ annimmt, und wenigstens ein Baustein (I), bei dem der Rest R¹ die von R⁵ oder R⁶ annimmt, und wenigstens ein Baustein (II) enthalten ist,
die Einheiten der allgemeinen Formel (III) proteinogene oder nicht proteinogene Aminosäuren sind und zu nicht mehr als 20 Gew.-%, bezogen auf copolymere Polyasparaginsäurederivate, enthalten sind.

Alle gegebenen Angaben zur Zusammensetzung der polymeren Produkte beziehen sich wie üblich auf die mittlere Zusammensetzung der Polymerketten.

Bevorzugt eingesetzt werden Produkte, in denen wenigstens ein Rest R¹ oder R² die Bedeutung von R⁵ annimmt.

Die restlichen Einheiten (max. 25 Mol.-% welche nicht die Struktur (I), (II) oder (III) haben) können unter anderem Iminodisuccinateinheiten der Struktur (IV) 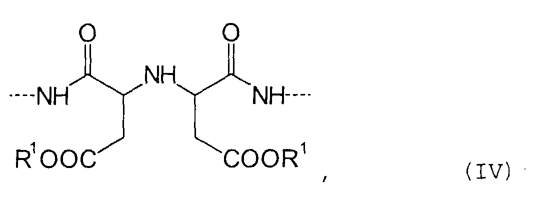 sowie verschiedene Endgruppen sein, am N-Terminus beispielsweise Asparaginsäure-, Maleinsäure-, Fumarsäure- und Apfelsäureeinheiten sowie deren Ester oder Amide, Maleinimideinheiten oder Diketopiperazine abgeleitet von Asparaginsäure und/oder den Aminosäurebausteinen (III), sowie Esser oder Amide der Aminosäurebausteine (III), am C-Terminus beispielsweise Asparaginsäure- oder Apfelsäureeinheiten, deren Mono- oder Diester, Amide oder cyclischen Imide.

Als Aminosäurebausteine (III) aus der Gruppe der proteinogenen Aminosäuren kommen z. B. Glutamin, Asparagin, Lysin, Alanin, Glycin, Tyrosin, Tryptophan, Serin und Cystein sowie deren Derivate in Frage; nicht proteinogene Aminosäuren können beispielsweise β-Alanin, ω-Amino-1-alkansäuren etc. sein.

Die Einheiten (II) leiten sich bevorzugt von primären oder sekundären Aminen NR²R³H ab, bei denen R² ein geradkettiger oder verzweigter, gesättigter, oder ungesättigter Alkyl-, Alkenyl- oder Arylrest mit 1 bis 30 C-Atomen, besonders bevorzugt ein Alkylrest mit 8 bis 24 C-Atomen (beispielsweise verzweigte oder lineare Octyl-, Decyl-, Dodecyl-, Tetradecyl-, Hexadecyl-, Octadecyl-, Docosylreste, auch ungesättigte und mehrfach ungesättigte Spezies wie beispielsweise Oleyl), und R³ Wasserstoff oder Methyl ist. Weiterhin bevorzugte Amine sind Aminoalkohole, beispielsweise Ethanolamin, Diethanolamin, Aminopropanol, deren N-Methylderivate sowie deren Acylierungsprodukte beispielsweise mit geradkettigen oder verzweigten, gesättigten, oder ungesättigten Carbonsäuren mit 1 bis 30 C-Atomen, besonders bevorzugt mit 8 bis 24 C-Atomen.

Eine bevorzugte Form der erfindungsgemäßen Copolymeren enthält wenigstens eine freie Carboxylatgruppe (R¹ = H, Metall, Ammonium), wenigstens einen Baustein (I), bei dem R¹ die Bedeutung von R⁵ oder R⁶ hat, sowie wenigstens einen Baustein (II), der als Rest R² einen Alkyl- oder Alkenylrest aus der Gruppe der geradkettigen oder verzweigten, gesättigten oder ungesättigten Alkylreste mit 8 bis 24 C-Atomen enthält (z. B. verzweigte oder lineare Octyl-, Decyl-, Dodecyl-, Tetradecyl-, Hexadecyl-, Octadecyl-, Docosylreste, auch ungesättigte und mehrfach ungesättigte Spezies wie z. B. Oleyl).

Eine weitere bevorzugte Form der erfindungsgemäßen Copolymeren enthält wenigstens eine freie Carboxylatgruppe (R¹ = H, Metall, Ammonium), wenigstens einen Baustein (I), bei dem R¹ die Bedeutung von R⁶ hat und R⁶ aus der Gruppe der geradkettigen oder verzweigten, gesättigten oder ungesättigten Alkyl- oder Alkenylreste mit 1 bis 5 C-Atomen stammt (z. B. Methyl, Ethyl, n-Propyl, i-Propyl, n-Butyl, i-Butyl, s-Butyl, n-Pentyl), sowie wenigstens einen Baustein (II), der als Rest R² einen Alkyl- oder Alkenylrest aus der Gruppe der geradkettigen oder verzweigten, gesättigten oder ungesättigten Alkyl- oder Alkenylreste mit 8 bis 24 C-Atomen enthält (beispielsweise verzweigte oder lineare Octyl-, Decyl-, Dodecyl-, Tetradecyl-, Hexadecyl-, Octadecyl-, Docosylreste, auch ungesättigte und mehrfach ungesättigte Spezies wie beispielsweise Oleyl). Besonders bevorzugt sind Verbindungen, bei denen R³ Wasserstoff oder Methyl ist.

Eine weitere bevorzugte Form der erfindungsgemäßen Copolymeren enthält wenigstens eine freie Carboxylatgruppe (R¹ = H, Metall, Ammonium), wenigstens einen Baustein (I), bei dem R¹ die Bedeutung von R⁵ hat und R⁵ für gleiche oder verschiedene, geradkettige oder verzweigte, gesättigte oder ungesättigte Alkyl- oder Alkenylreste R¹¹ mit 6 bis 30 C-Atomen oder Radikale der Struktur -Y-R¹¹, wobei Y eine Oligo- oder Polyoxyalkylenkette mit 1 bis 100 Oxyalkyleneinheiten ist, sowie wenigstens einen Baustein (II), der als Rest R² einen Alkyl- oder Alkenylrest aus der Gruppe der geradkettigen oder verzweigten, gesättigten oder ungesättigten Alkyl- oder Alkenylreste mit 1 bis 30 C-Atomen, besonders bevorzugt 1 bis 8 C-Atomen, enthält. Besonders bevorzugt sind Verbindungen mit Alkyl- oder Alkenylresten R⁵ ohne Alkylenglykolspacer und mit Resten R³, die Wasserstoff oder Methyl sind.

Die erfindungsgemäßen Copolymeren lassen sich beispielsweise dadurch erhalten, daß man ein Gemisch von Monoestern und Monoamiden, optional in Gegenwart von Diestern und/oder Diamiden und/oder Anhydriden, monoethylenisch ungesättigter Dicarbonsäuren mit 0,5-1,5 Äquivalenten Ammoniak umsetzt bzw. die Ammoniumsalze dieser Säuren thermisch in das Polymer überführt. Eingesetzt werden können beispielsweise Derivate der Maleinsäure, Fumarsäure, Itaconsäure, Alkenylbernsteinsäure, Alkylmaleinsäure, Citraconsäure oder deren Ammoniumsalze, vorzugsweise Derivate der Maleinsäure, Fumarsäure oder Itaconsäure, besonders vorzugsweise Maleinsäurederivate der allgemeinen Formeln (V), (VI) und (VII) 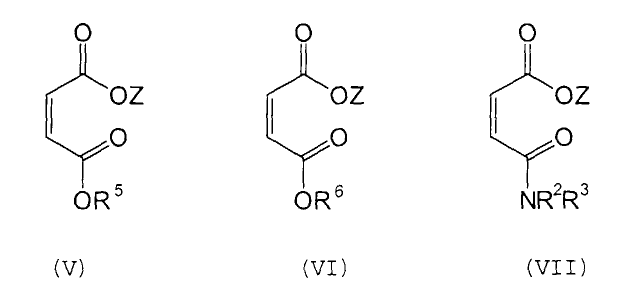 wobei Z für Wasserstoff oder Ammonium, R², R³, R⁵ und R⁶ für die oben genannten Reste stehen und der Anteil an (VII) nicht gleich 0 ist ebenso wie die Summe der Anteile aus (V) und (VI). Eingesetzt werden Mischungen mit einem Gesamtanteil von 0.5 bis 99 Gew.-%, bevorzugt 10 bis 90 Gew.-%, besonders bevorzugt 40 bis 95 Gew.-%, an Esterkomponente (V) und/oder (VI) sowie mit 1 bis 100 Gew.-%, bevorzugt 10 bis 90 Gew.-%, besonders bevorzugt 5 bis 60 Gew.% an Amidkomponente (VII).

Vorzugsweise eingesetzte Reste R⁵ sind Alkyl- oder Alkenylreste mit 8 bis 30 C-Atomen, beispielsweise lineare oder verzweigte Decyl-, Dodecyl-, Tetradecyl-, Hexadecyl-, oder Octadecylreste sowie ungesättigte Alkyl- oder Alkenylreste, wie z. B. Oleyl. Vorzugsweise eingesetzte Reste R⁴ sind Alkylreste mit 1 bis 4 C-Atomen, vorzugsweise Methyl, Ethyl, n-Propyl, i-Propyl, n-Butyl, i-Butyl, s-Butyl.

Die Reaktion kann mit oder ohne Zusatz von organischen Lösungsmitteln erfolgen. Als Lösungsmittel kommen beispielsweise Alkohole, Ketone, Ester, Oligo- und Poly(alkylen)glykole bzw. - glykolether, Dimethylsulfoxid, Dimethylformamid, N,N-Dimethylacetamid oder N-Methylpyrrolidon sowie deren Gemische und andere in Frage. Bevorzugt eingesetzt werden Alkohole mit 2-4 C-Atomen, davon besonders bevorzugt der kurzkettige Alkohol R⁴OH, sowie Ketone wie beispielsweise Methylisobutylketon oder Methylisoamylketon oder Alkylester von Carbonsäuren mit 1-4 C-Atomen, wie beispielsweise Essigsäure-sec-Butylester oder Essigsäurepentylester.

Die Reaktion kann optional in Gegenwart von verträglichkeitsfördernden Agenzien durchgeführt werden. Dieses können grenzflächenaktive Verbindungen sein, beispielsweise Anlagerungsprodukte von 1 bis 30 Mol Ethylenoxid und /oder 0 bis 5 Mol Propylenoxid an C₁₂-C₃₀-Fettalkohole und Wollwachsalkohole; Ethylenoxidanlagerungsprodukte von Glycerinmono- und -diestern und Sorbitanmono- und diestern von gesättigten und ungesättigten Fettsäuren mit 6 bis 22 C-Atomen; Anlagerungsprodukte von 2 bis 30 Mol Ethylenoxid und / oder 0 bis 5 Mol Propylenoxid an Fettsäuren mit 12 bis 22 C-Atomen und an Alkylphenole mir 8 bis 15 C-Atomen in der Alkylgruppe; C₁₂-C₁₈-Fettsäurepartialesser von Anlagerungsprodukten von 1 bis 30 Mol Ethylenoxid an Glycerin; Anlagerungsprodukte von Ethylenoxid an Fette und Öle, beispielsweise Rizinusöl oder gehärtetes Rizinusöl; Partialester von gesättigten oder ungesättigten C₁₂-C₂₂-Fettsäuren, auch verzweigte oder hydroxysubstituierte, mit Polyolen, beispielsweise Ester von Glycerin, Ethylenglykol, Polyalkylenglykolen, Pentaerythrit, Polyglyrerin, Zuckeralkoholen wie Sorbit und Polyglucosiden wie Cellulose; Polysiloxan-Polyalkyl-Polyether-Copolymere und deren Derivate sowie hydrophob modifizierte Polyasparaginsäurederivate, beispielsweise teilveresterte Polyasparaginsäuren, teilveresterte Polyasparaginsäure-co-Glutaminsäuren oder Kondensate aus Maleinsäuremonoestern und Ammoniak, beispielsweise hergestellt nach dem erfindungsgemäßen Verfahren oder nach DE 195 45 678 A, wobei das Herstellverfahren der genannten Polyaminosäurederivate keinen Einfluß auf deren verträglichkeitsvermittelnde Wirkung hat. Gegebenenfalls kann auch ein gewisser Teil der Produktmischung im Reaktor verbleiben und als Lösungsvermittler für eine folgende Umsetzung dienen.

Als verträglichkeits- bzw. löslichkeitsvermittelnde Agenzien können auch kationische Tenside, beispielsweise aus der Gruppe der quarternären Ammoniumverbindungen, quarternisierten Proteinhydrolysare, Alkylamidoamine, quarternären Esterverbindungen, quarternären Siliconöle oder quarternären Zucker- und Polysaccharidderivate, anionische Tenside beispielsweise aus der Gruppe der Sulfate, Sulfonate, Carboxylate sowie Mischungen derselben, beispielsweise Alkylbenzolsulfonate, α-Olefinsulfonate, α-sulfonierte Fettsäureester, Fettsäureglycerinestersulfate, Paraffinsulfonate, Alkylsulfate, Alkylpolyethersulfate, Sulfobernsteinsäurealkylester, Fettsäuresalze (Seifen), Fettsäureester der Polymilchsäure, N-Acylaminosäureester, N-Acyltaurate, Acylisethionate, Ethercarboxylate, Monoalkylphosphate, N-Acylaminosäurederivate wie N-Acylaspartate oder N-Acylglutamate, N-Acylsarcosinate, amphotere oder zwitterionische Tenside wie beispielsweise Alkylbetaine, Alkylamidoalkylbetaine des Typs Cocoamidopropylbetain, Sulfobetaine, Phosphobetaine, Sultaine und Amidosultaine, Imidazoliniumderivate, Amphoglycinate, oder nichtionische Tenside wie beispielsweise oxethylierte Fettalkohole, oxethylierte Alkylphenole, oxethylierte Fettsäureester, oxethylierte Mono-, Di- oder Triglyceride oder Polyalkylenglykolfettsäureester, Zuckerester, z.B. Fettsäureester der Saccharose, Fructose oder des Methylglucosids, Sorbitolfettsäureester und Sorbitanfettsäureester (gegebenenfalls oxethyliert), Alkyl- oder Alkenylpolyglucoside und deren Ethoxylate, Fettsäure-N-alkylpolyhydroxyalkylamide, Polyglycerinester, Fettsäurealkanolamide, langkettige tertiäre Aminoxide oder Phosphinoxide sowie Dialkylsulfoxide enthalten sein.

Vorzugsweise verbleiben die verträglichkeitsfördernden Agenzien im Produkt. Die Umsetzung zum Copolymeren erfolgt in einer bevorzugten Ausführungsweise mit wäßrigem oder gasförmigen Ammoniak bei Temperaturen von 0 bis 150°C, vorzugsweise 50-140°C sowie nachfolgendem Ausdestillieren bei 70 bis 240°C, vorzugsweise 110 bis 150°C, unter vermindertem Druck, beispielsweise in Knetapparaturen, Hochviskoreaktoren, Extrudern und Rührreaktoren, gegebenenfalls unter Einsatz scherkraftreicher Rührer wie Mig- oder Intermig-Rührer.

Unter den Reaktionsbedingungen werden gleichzeitig ein Teil der Estergruppen, bevorzugt die von R⁶OH abgeleiteten, hydrolysiert und die gewünschten Carbonsäure- bzw. Carboxylatgruppen freigesetzt. Durch nachfolgende milde partielle oder vollständige Hydrolyse, bevorzugt der vom kurzkettigen Alkohol R⁶OH abgeleiteten Esterfunktionen kann, wenn gewünscht, der Anteil freier Säuregruppen weiter erhöht werden, beispielsweise durch Umsetzung mit Wasser ggf. in Gegenwart von Säuren oder Basen, oder mit Alkalimetallhydroxiden, ggf. in Gegenwart eines organischen Solvens oder Cosolvens.

Die Ester- und Amidkomponente können jeweils beliebige Mischungen aus Verbindungen mit unterschiedlichen Resten R², R³, R⁵, bzw. R⁶ sein.

Anstelle von Maleinsäuremonoester und -amiden kann auch direkt Maleinsäureanhydrid im Gemisch mit den entsprechenden Alkoholen und Aminen eingesetzt werden.

Durch Zusatz von amino- und carboxyfunktionellen Verbindungen zur Reaktionsmischung können Copolymere erhalten werden, in denen die angebotenen Bausteine über Amidbindungen gebunden vorliegen. Geeignete Bausteine sind Aminosäuren aus der Gruppe der 20 proteinogenen Aminosäuren, welche als Monomere in allen natürlichen Proteinen enthalten sind, in enantiomerenreiner oder racemischer Form, wie beispielsweise Glutaminsäure, Glutamin, Asparagin, Lysin, Alanin, Glycin, Tyrosin, Tryptophan, Serin und Cystein sowie deren Derivate, oder nicht proteinogenen Aminosäuren mit jeweils einer oder mehreren Amino- bzw. Carboxyfunktionen, wie beispielsweise β-Alanin, ω-Amino-1-alkansäuren, beispielsweise 6-Aminocapronsäure. Die Bausteine, vorzugsweise 0 bis 15 Gew.-%, werden der Ausgangsmischung der Maleinsäurederivate zugesetzt oder zur Modifizierung der Kettenenden nach erfolgter Synthese des Polymeren mit diesem umgesetzt, vorzugsweise unter Zusatz polarer Solventien, wie beispielsweise Alkoholen oder Dimethylformamid.

Die Molekularmasse der Polyasparaginsäurederivate kann durch Zusatz von di- und/oder polyfunktionellen Bausteinen, abgeleitet von einer Di- oder Polyhdroxyverbindung, einer Di- oder Polyaminoverbindung, oder Aminoalkoholen mit einem linearen, verzweigten oder cyclischen, gesättigten , ungesättigten oder aromatischen Kohlenwasserstoffgerüst, ggf. oxo- oder aza-Analogen mit O- oder N-Atomen in der Kette, oder von Polyalkylenglykolen bzw. Ethylenoxid-Propylenoxid-Copolymeren, erhöht werden. Die Einführung der molekularmassenerhöhenden Gruppen erfolgt durch Zusatz der polyfunktionellen Amino- bzw. Hydroxyverbindungen bzw. deren Umsetzungsprodukten mit Maleinsäureanhydrid zur Reaktionsmischung oder zum gebildeten Polymeren, ggf. unter Zusatz von sauren oder lewissauren Katalysatoren.

Die beschriebenen Vorgehensweisen können auch kombiniert werden.

Die resultierenden Polymeren können nachbehandelt werden, beispielsweise durch Behandlung mit Ammoniak, Umesterungskatalysatoren wie beispielsweise lewissauren Titan-(IV)-Verbindungen, mit Aktivkohle oder anderen Adsorbentien, Bleichung mit Oxidationsmitteln wie H₂O₂, Cl₂, O₃, Natriumchlorit, Natriumhypochlorit etc. oder Reduktionsmitteln wie beispielsweise NaBH₄ oder H₂ in Gegenwart von Katalysatoren, unter üblichen Bedingungen.

Die erfindungsgemäßen Copolymeren besitzen hervorragende Eigenschaften als Sequestriermittel, als Additive zu Farben und Lacken, als Schaumstabilisatoren, Tenside und Emulgatoren. Insbesondere die Temperatur- und Langzeitstabilität von O/W- und W/O-Emulsionen wird positiv beeinflußt.

Die erfindungsgemäßen Polymeren können als O/W-Emulgatoren für kosmetische Emulsionen eingesetzt werden, beispielsweise für Lotionen mit einer vergleichsweise niedrigen Viskosität oder Cremes und Salben mit einer hohen Viskosität, für Anwendungen als Hautpflegemittel wie beispielsweise Tagescremes, Nachtcremes, Pflegecremes, Nährcremes, Bodylotions, Salben und dergleichen. Als weitere Hilfs- und Zusatzstoffe können übliche Coemulgatoren, Konsistenzgeber, Ölkörper, Überfettungsmittel, Fette, Wachse, Stabilisatoren, Wirkstoffe, Glycerin, Farb- und Duftstoffe enthalten.

Als Konsistenzgeber können hydrophile Wachse, beispielsweise C₁₂-C₃₀-Fetralkohole, C₁₆-C₂₂-Fettsäuren, Glycerinmono- und -diester und Sorbitanmono- und diester von gesättigten Fettsäuren mit 12 bis 22 C-Atomen eingesetzt werden.

Als weitere Coemulgatoren kommen beispielsweise in Frage: Anlagerungsprodukte von 2 bis 30 Mol Ethylenoxid und /oder 0 bis 5 Mol Propylenoxid an C₁₂-C₃₀-Fettalkohole und Wollwachsalkohole, vorzugsweise lineare, gesättigte C₁₆-C₂₂- Fettalkohole; Ethylenoxidanlagerungsprodukte von Glycerinmono- und - diestern und Sorbitanmono- und diestern von gesättigten und ungesättigten Fettsäuren mit 6 bis 22 C-Atomen; Anlagerungsprodukte von 2 bis 30 Mol Ethylenoxid und /oder 0 bis 5 Mol Propylenoxid an Fettsäuren mit 12 bis 22 C-Atomen und an Alkylphenole mit 8 bis 15 C-Atomen in der Alkylgruppe; C₁₂-C₁₈-Fettsäurepartialester von Anlagerungsprodukten von 1 bis 30 Mol Ethylenoxid an Glycerin; Anlagerungsprodukte von Ethylenoxid an Fette und Öle, beispielsweise Rizinusöl oder gehärtetes Rizinusöl; Partialester von gesättigten oder ungesättigten C₁₂-C₂₂-Fettsäuren, auch verzweigte oder hydroxysubstituierte, mit Polyolen, beispielsweise Ester von Glycerin, Ethylenglykol, Polyalkylenglykolen, Pentaerythrit, Polyglycerin, Zuckeralkoholen wie Sorbit und Polyglucosiden wie Cellulose; Polysiloxan-Polyalkyl-Polyether-Copolymere und deren Derivate sowie hydrophob modifizierte Polyasparaginsäurederivate.

Als Coemulgatoren können auch anionische, kationische, nichtionische, amphotere und/oder zwitterionische Tenside, beispielsweise aus der als verträglichkeitsfördernden Agenzien bezeichneten Gruppe ausgewählt sein.

Es können jeweils beliebige Mischungen der o.g. Konsistenzgeber und Coemulgatoren eingesetzt werden.

Als Ölkörper kommen beispielsweise Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₂₀-Fettsäuren mit verzweigten Alkoholen, Ester von linearen und/oder verzweigten C₆-C₂₀-Carbonsäuren mit mehrwertigen Alkoholen und/oder Guerbetalkoholen, Triglyceride auf Basis von C₆-C₁₀-Fettsäuren, pflanzliche und tierische Öle und Fette, verzweigte primäre Alkohole, substituierte Cyclohexane, Guerbetcarbonate, Dialkylether und/oder aliphatische bzw. naphthenische Kohlenwasserstoffe in Betracht.

Als Überfettungsmittel können beispielsweise Lanolin und Lecithinderivate sowie deren Ethoxylate, Polyolfettsäureester, Monoglyceride und Fettsäurealkanolamide verwendet werden. Es können Siliconverbindungen wie Polydimethylsiloxane, Cyclodimethicone sowie amino-, fettsäure-, alkohol- epoxy-, fluor-, und/oder alkylmodifizierte Siliconverbindungen sowie Wachse wie beispielsweise Bienenwachs, Paraffinwachse oder Mikrowachse enthalten sein. Die Emulsionen können Verdickungsmittel wie Polyacrylsäurederivate oder kationische Polymere wie z.B. kationische Cellulose- oder Stärkederivate, kationische Chitin oder Chitosanderivate, kationische Siliconpolymere, Copolymere von Diallylammoniumsalzen beispielsweise mit Acrylamiden, Polyethylenimin enthalten. Weiterhin können Metallsalze von Fettsäuren, beispielsweise Magnesium-, Aluminium- oder Zinkstearat als Stabilisatoren oder Zinksalze der Ricinolsäure als Geruchshemer enthalten sein. Es können übliche Sonnenschutzwirkstoffe wie Titandioxid, p-Aminobenzoesäure etc., Duftstoffe, Farbstoffe, biogene Wirkstoffe wie Pflanzenextrakte oder Vitaminkomplexe sowie pharmazeutische Wirkstoffe enthalten sein. Weiterhin können die Emulsionen Perlglanzmittel wie Ethylenglykoldistearat sowie die üblichen Konservierungsmittel wie Parabene, Sorbinsäure, Phenoxyethanol und andere enthalten.

Die erfindungsgemäßen Polyasparaginsäurederivate können auch in W/O-Emulsionen eingesetzt werden, beispielsweise als Emulgatoren und/oder Coemulgatoren für die Herstellung von Hautpflegecremes und -lotionen.

Die erfindungsgemäßen Polyasparaginsäurederivate mit einem naturnahen Polyaminosäurerückgrat sind milde Tenside, welche alleine oder in Kombination mit anionischen, kationischen, nichtionischen, zwitterionischen und/oder amphoteren Tensiden eingesetzt werden können. Es sind feste, flüssige oder pastöse Zubereitungen möglich, z.B. Seifenstücke, Waschlotionen, Duschgele, Shampoos.

Die in Kombination mit den erfindungsgemäßen Polyasparaginsäurederivaten einsetzbaren Tenside können beispielsweise anionische Tenside aus der Gruppe der Sulfate, Sulfonate, Carboxylate sowie Mischungen derselben sein. Die anionischen Gruppen können in neutralisierter Form vorliegen, mit kationischen Gegenionen aus der Gruppe der Alkalimetalle, Erdalkalimetalle, Ammonium oder substituiertem Ammonium. Eingesetzt werden beispielsweise Alkylbenzolsulfonate, α-Olefinsulfonate, α-sulfonierte Fettsäureester, Fettsäureglycerinestersulfate, Paraffinsulfonate, Alkylsulfate, Alkylpolyethersulfate, Sulfobernsteinsäurealkylester, Fettsäuresalze (Seifen), Fettsäureester der Polymlichsäure, N-Acylaminosäureester, N-Acyltaurate, Acylisethionate, Ethercarboxylate, Monoalkylphosphate, N-Acylaminosäurederivate wie N-Acylaspartate oder N-Acylglutamate, N-Acylsarco-sinate, Polyasparaginsäurederivate und andere.

Die in Kombination mit den erfindungsgemäßen Polyasparaginsäurederivaten einsetzbaren Tenside können beispielsweise amphotere oder zwitterionische Tenside sein, beispielsweise Alkylbetaine, Alkylamidoalkylbetaine des Typs Cocoamidopropylbetain, Sulfobetaine, Phosphobetaine, Sultaine und Amidosultaine, Imidazoliniumderivate, Amphoglycinate und andere.

Die in Kombination mit den erfindungsgemäßen Polyasparaginsäurederivaten einsetzbaren kationischen Tenside können beispielsweise aus der Gruppe der quarternären Ammoniumverbindungen, quarternisierten Proteinhydrolysate, Alkylamidoamine, quarternären Esterverbindungen, quarternären Siliconöle oder quarternären Zucker- und Polsaccharidderivate ausgewählt sein.

Die in Kombination mit den erfindungsgemäßen Polyasparaginsäurederivaten einsetzbaren Tenside können beispielsweise nichtionische Tenside sein, beispielsweise oxethylierte Fettalkohole, oxethylierte Alkylphenole, oxethylierte Fettsäureester, oxethylierte Mono-, Di- oder Triglyceride oder Polyalkylenglykolfettsäureester. Andere nichtionische Tenside können aus der Gruppe der Alkylpolysaccharide, beispielsweise Alkyl- oder Alkenylpolyglucoside, Zuckerester, beispielsweise Fettsäureester der Glucose, Saccharose, Fructose oder des Methylglucosids, Sorbitolfettsäureester und Sorbitanfettsäureester (gegebenenfalls oxethyliert), Polyglycerinester, Fettsäurealkanolamide, N-Acylaminozuckerderivate beispielsweise N-Acylglucamine, langkettige tertiäre Aminooxide oder Phosphinoxide sowie Dialkylsulfoxide stammen.

Die in Kombination mit den erfindungsgemäßen Polyasparaginsäurederivaten eingesetzten Tenside können somit auch beliebige Kombinationen aus zwei oder mehr Tensiden der obengenannten Kategorien sein.

Die erfindungsgemäßen Tensidzubereitungen können weitere Hilfs- und Zusatzstoffe enthalten wie beispielsweise Wasser und Lösungsmittel beispielsweise aus der Gruppe der Alkohole und Polyole, Verdickungsmittel, Trübungsmittel, z.B. Glykolesterderivate; Moisturizer, Emollients wie tierische und pflanzliche Öle, Carbonsäureester, Lanolin, Bienenwachs, Silicone; polymere Agenzien zur Verbesserung des Hautgefühls, konditionierende, pflegende oder pharmazeutisch wirksame Bestandteile wie beispielsweise kationische oder amphotere Polymere, Proteine und Proteinderivate, Lanolinderivate, Panthothensäure, Betain, Polydimethylsiloxane oder deren Derivate, Sonnenschutzwirkstoffe sowie Lösungsvermittler, Stabilisatoren, Geruchsstoffe, Puffersubstanzen, Konservierungsmittel und/oder Farbstoffe.

Die Polyasparaginsäurederivate enthaltenden Tensidzubereitungen lassen sich vorteilhaft anwenden in beispielsweise Haarshampoos, Duschbäder, Schaumbadzubereitungen, Hand-, Gesichts und Intimreinigungslotionen, Flüssigseifen, Seifenstücke, Rasiercremes, Handwaschpasten, hautfreundlichen Geschirrspülmitteln, Reinigungsmitteln für glatte Oberflächen sowie in Zahncremes.

Die erfindungsgemäßen Polyasparaginsäurederivate können als Dispergiermittel beispielsweise für Lacke und Farben eingesetzt werden.

Die erfindungsgemäßen hydrophob modifizierten Polyasparaginsäurederivate werden dazu vorteilhaft mit dem Stand der Technik entsprechenden Neutralisationsmitteln, insbesondere Aminen neutralisiert sofern nicht bereits als Salz vorliegend. Insbesondere bevorzugt ist hier die Verwendung von Dimethylethanolamin oder 2-Amino-2-methylpropanol. Zur Herstellung wäßriger Pigmentpasten werden 0.1-100 Gew.-%, vorzugsweise 0.5-50 Gew.-%, insbesondere 2 bis 15 Gew.-% bezogen auf das Gewicht der Pigmente verwendet. Die hydrophob modifizierten Polyasparaginsäurederivate können bei der erfindungsgemäßen Verwendung entweder vorab mit den zu dispergierenden Pigmenten vermischt werden oder direkt in dem Dispergiermedium (Wasser, eventuelle Glycolzusätze) vor oder gleichzeitig mit der Zugabe der Pigmente und etwaiger anderer Feststoffe gelöst werden. Die Neutralisation kann dabei vor oder während der Herstellung der Pigmentpasten erfolgen. Bevorzugt werden Polyasparaginsäurezubereitungen eingesetzt, welche bereits partiell oder vollständig neutralisiert werden.

Die erfindungsgemäßen Polyasparaginsäurederivate können auch in beliebigen Gemischen mit weiteren, dem Stand der Technik entsprechenden Dispergieradditiven, beispielsweise aus der Gruppe der Fettsäurealkoxylate, Poly(meth)acrylate, Polyester, Polyether etc., eingesetzt werden.

Als Pigmente können in diesem Zusammenhang beispielsweise anorganische oder organische Pigmente, sowie Ruße genannt werden. Als anorganische Pigmente seien exemplarisch genannt Titandioxid und Eisenoxide. In Betracht zu ziehende organische Pigmente sind beispielsweise Azopigmente, Metallkomplex-Pigmente, Phthalocyaninpigmente, anthrachinoide Pigmente, polycyclische Pigmente, insbesondere solche der Thioindigo-, Chinacridon-, Dioxazin-, Pyrrolopyrrol-, Naphthalintetracarbonsäure-, Perylen-, Isoamidolin(on)-, Flavanthron-, Pyranthron- oder Isoviolanthron-Reihe.

Füllstoffe, die beispielsweise in wäßrigen Lacken dispergiert werden können, sind beispielsweise solche auf Basis von Kaolin, Talkum, anderen Silikaten, Kreide, Glasfasern, Glasperlen oder Metallpulvern.

Als Lacksysteme, in denen die erfindungsgemäßen Pigmentpasten aufgelackt werden können, kommen beliebige wäßrige 1K- oder 2K-Lacke in Betracht. Beispielhaft genannt seien wäßrige 1K-Lacke wie beispielsweise solche auf Basis von Alkyd-, Acrylat-, Epoxid-, Polyvinylacetat-, Polyester- oder Polyurethanharzen oder wäßrige 2K-Lacke, beispielsweise solchen auf Basis von hydroxylgruppenhaltigen Polyacrylat- oder Polyesterharzen mit Melaminharzen oder gegebenenfalls blockierten Polyisocyanatharzen als Vernetzer. In gleicher Weise seien auch Polyepoxidharzsysteme genannt.

Die erfindungsgemäßen Polyasparaginsäurederivate können als komplexbildende Agenzien, beispielsweise in Waschmitteln, als Inkrustationsinhibitoren, als Metalldesaktivatoren in Kunststoffen, als Hilfsstoffe in der Papier-, Leder- und Textilindustrie oder als wirkungsverstärkende Zusatzstoffe zu Pestiziden oder Insektiziden eingesetzt werden. Hochmolekulare Derivate, vorzugsweise nach Modifizierung mit den obengenannten polyfunktionellen Hydroxy- und Aminoverbindungen, eignen sich auch als Absorbermaterialien.

### Beispiele

### Beispiele 1 bis 6

Die Edukte (Monoethylmaleat, Monoalkylmaleat, N-Alkylmaleamid gemäß Tab. 1, gelöst in 4-Methyl-2-pentanon)wurden mit 1.0 bis 1.5 Äquivalenten an Ammoniakgas umgesetzt und die Reaktionsmischung im Vakuum bei 110 °C bis 140 °C für 5 h ausdestilliert.

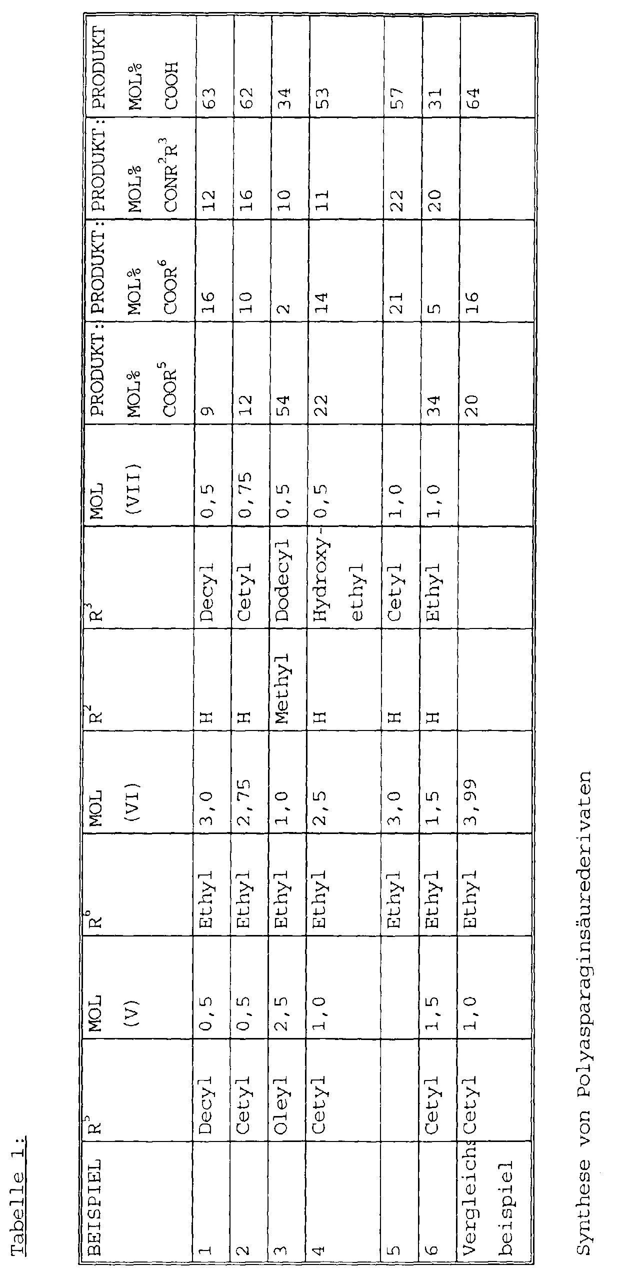

### Beispiel 7

### O/W-Emulsion mit Polyasparaginsäurederivaten

| | | |
|---|---|---|
| Cetylpolyaspartat aus Beispiel 2 (25 % in Wasser, pH 5.5) | 2,0 % | |
| | Glycerin | 3,0 % |
| Konservierungsmittel | 0,1 % | |
| Wasser | 70,0 % | |
| Glycerinmonostearat (Tegin® M, Th. Goldschmidt) | 4,5 % | |
| Tegosoft® CT (Capryl-Caprintriglycerid, Th. Goldschmidt) | 20,00 % | |

Die wäßrige Phase und die Ölkörper/Glycerinmonostearatmischung wurden bei 70°C zusammengegeben, intensiv mit einem Rotor-Stator-Homogenisator bearbeitet (SG/220V, 2 min). Die Emulsion (100 ml) wurde 2 Tage bei 20°C und 7 d bei 45°C gelagert. Die sensorische Bewertung der Proben zeigte bei Beispiel 7 keine Änderung der cremeartigen Konsistenz, beim Vergleichsbeispiel 2 einen Viskositätsverlust. Die Wasserseparation der W/O-Emulsionen wurde nach 2 Tagen Lagerung bei 20 °C und nach weiteren 7 Tagen Lagerung bei 45°C bestimmt.

| BEISPIEL | EMULGATOR AUS BEISPIEL | WASSERSEPAPATION NACH 2 TAGEN/20°C (VOL %) | WASSERSEPARATION NACH 28 TAGEN/45°C (VOL%) |
|---|---|---|---|
| 7 | 2 | < 0,1 % | < 0,1 % |
| Vergleichsbeispiel 2 | Vergleichsbei spiel 2 | < 0,1 % | 1,0 % |

Diese Ergebnisse zeigen die erhöhte Emulsionsstabilität bei den amidmodifizierten Polyasparaginsäureestern.

### Beispiel 8

### Schäumende Tensidzubereitung mit Polyasparaginsäurederivaten:

| Rezeptur | (A) | (B) |
|---|---|---|
| | [Gew%] | |
| Produkt nach Beispiel 1 (50 %ig in Wasser, pH 5,5) | 0,0 % | 1,0 % |
| Texapon® N28 (28 % Natriumlaurylethersultat, Henkel) | 21,4 % | 21,4 % |
| Tego® Betain F50 (37,5 % Cocoamidopropylbetain, Th. Goldschmidt) Wasser ad 100 %, pH ad 6,0 | 16,0 % | 16,0 % |

Die Schaumeigenschaften der Tensidmischung wurden durch Aufschäumen einer verdünnten Tensidlösung bestimmt. (0.5 Gew% WAS, 8°dH, 30°C, Ystral-Leitstrahlmischer, 750 W, 2 min)

| MISCHUNG | SCHAUMVOLUMEN [ML] | WASSERSEPAPATION 10 MIN [ML] | SCHAUMDICHTE [G/ML] |
|---|---|---|---|
| A | 1490 ± 17 | 240 ± 2.0 | 0.208 ± 0.002 |
| B | 1573 ± 10 | 236 ± 2.9 | 0.191 ± 0.003 |

Dieses Beispiel belegt den positiven Einfluß der Polyasparaginsäurederivate auf das Schaumverhalten von Tensidsystemen.

### Beispiel 9

| | |
|---|---|
| Polyasparaginsäurederivat nach Beispiel 1, (50 %ig in Wasser, pH 5,5) | 11,0 % |
| Texapon® N70 (70 % Natriumlaurylethersulfat, Henkel) | 32,0 % |
| Tagat® R40 (PEG-40-Ethoxylat von hydriertem Rizinusöl, Th. Goldschmidt) | 5,0 % |
| Tego® Glucosid 810 (60 % Capryl/Capringlucosid, Th. Goldschmidt) | 8,0 % |
| Zitronensäure (20 %) | 0,9 % |
| NaCl (25 %) | 8,5 % |
| Wasser | 16,6 % |
| Tego® Betain F50 (37,5 % Cocoamidopropylbetain, Th. Goldschmidt) | 18,0 % |

### Beispiel 10

### Pflegecreme auf O/W-Basis

| | |
|---|---|
| Polyasparaginsäurederivat nach Beispiel 2, (50 %ig in Wasser pH 5,5) | 4,0 % |
| Polyasparaginsäurederivat nach Beispiel 4, (50 %ig in Wasser, pH 5,5) | 1,0 % |
| Tego® Care 450 (Polyglyceryl-3-methylglucosiddistearat, Th. Goldschmidt) | 1,0 % |
| Tegin® M (Glycerylstearat, Th. Goldschmidt) | 0,5 % |
| Tego® Akanol 18 (Stearylalkohol, Th. Goldschmidt) | 0,3 % |
| Avocadoöl | 12,0 % |
| Tegosoft® CT (Capryl-Caprintriglycerid, Th. Goldschmidt) | 9,0 % |
| Glycerin | 3,0 % |
| Wasser | 69,2 % |
| NaOH (10 %) ad pH 5,5 | |

### Beispiel 11

### W/O-Creme

| | |
|---|---|
| Polyasparaginsäurederivat nach Beispiel 3 (50 %ig in Wasser, pH 5,5) | 3,0 % |
| Abil® EM90 (Cetylpolyethersiloxan, Th. Goldschmidt) | 1,5 % |
| Isolan® GI 34 (Polyglyceryl-4-Isostearat, Th. Goldschmidt) | 0,8 % |
| Avocadoöl | 11,0 % |
| Tegosoft® CT (Capryl-Caprintriglycerid, Th. Goldschmidt) | 11,0 % |
| hydriertes Rizinusöl | 0,8 % |
| Bienenwachs | 1,0 % |
| NaCl | 0,5 % |
| Wasser | 0,5 % |
| Duftstoffe, Konservierungsmittel | 70,4 % |

## Patentansprüche

1. Von Polyaminosäuren abgeleitete Copolymere, die zu mindestens 75 Mol.-% der vorhandenen Einheiten aus Struktureinheiten der allgemeinen Formeln (I), (II) oder (III) 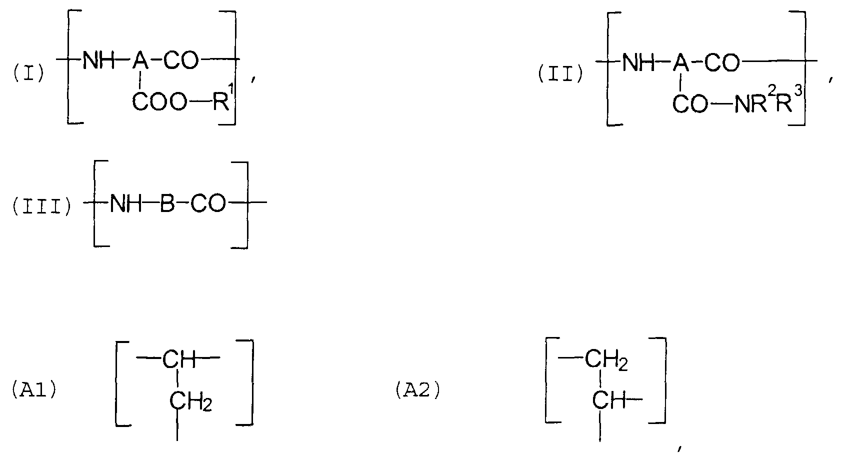 bestehen, in denen die Strukturelemente A gleiche oder verschiedene trifunktionelle Kohlenwasserstoffradikale mit 2 C-Atomen des Typs (A1) oder (A2) sind, worin
R¹ die Bedeutung von R⁴, R⁵, und R⁶ haben kann,
R² für gleiche oder verschiedene, geradkettige oder verzweigte, gesättigte oder ungesättigte Alkyl-, Alkenyl- oder Arylreste mit 1 bis 30 C-Atomen, für Hydroxy- oder Aminoalkylreste mit 1 bis 22 C-Atomen und 1 bis 6 Hydroxy- und/oder Aminogruppen und/oder deren Acylierungsprodukte mit 1 bis 22 C-Carbonsäuren oder die Bedeutung von R⁵ annimmt, und
R³ gleich Wasserstoff oder R² ist,
R⁴ für ein oder mehrere Reste aus der Gruppe der Alkali-, Erdalkalimetalle, Wasserstoff oder Ammonium, [NR⁷R⁸R⁹R¹⁰] + , worin
R⁷ bis R¹⁰ unabhängig voneinander Wasserstoff, Alkyl oder Alkenyl mit 1 bis 22 C-Atomen oder Hydroxyalkyl mit 1 bis 22 C-Atomen ist, mit 1 bis 6 Hydroxygruppen.
R⁵ für gleiche oder verschiedene, geradkettige oder verzweigte, gesättigte oder ungesättigte Alkyl- oder Alkenylreste R¹¹ mit 6 bis 30 C-Atomen oder Radikale der Struktur -Y-R¹¹, wobei Y eine Oligo- oder Polyoxyalkylenkette mit 1 bis 100 Oxyalkyleneinheiten ist, und
R⁶ für gleiche oder verschiedene, geradkettige oder verzweigte, gesättigte oder ungesättigte Alkyl- oder Alkenylreste mit 1 bis 5 C-Atomen steht,
und wenigstens jeweils eine Einheit der allgemeinen Formel (I), bei dem der Rest R¹ die Bedeutung von R⁴ annimmt, und wenigstens eine Einheit der allgemeinen Formel (II) enthalten ist, und die Einheiten der allgemeinen Formel (III) proteinogene oder nicht proteinogene Aminosäuren sind und zu nicht mehr als 20 Gew.-%, bezogen auf copolymere Polyasparaginsäurederivate, enthalten sind.

2. Copolymere nach Anspruch 1, in denen mindestens ein Rest R¹ die Bedeutung von R⁵ oder R⁶ annimmt.

3. Copolymere nach Anspruch 1 oder 2, in denen mindestens ein Rest R¹ oder R² die Bedeutung von R⁵ annimmt.

4. Copolymere nach einem der Ansprüche 1 bis 3, bei denen sich die Strukturelemente II von primären oder sekundären Aminen NR²R³H ableiten, bei denen R² ein geradkettiger oder verzweigter, gesättigter, oder ungesättigter Alkyl-, Alkenyl- oder Arylrest mit 1 bis 30 C-Atomen, und R³ Wasserstoff oder Methyl ist.

5. Copolymere nach einem der Ansprüche 1 bis 3, bei denen sich die Strukturelemente (II) von Aminoalkoholen wie Ethanolamin, Diethanolamin, und/oder Aminopropanolen ableiten sowie von deren N-Methylderivaten oder deren Acylierungsprodukten.

6. Copolymere nach einem der Ansprüche 1 bis 5, welche in Gegenwart von molekularmassenerhöhenden Agenzien aus der Gruppe der Di- oder Polyhydroxyverbindungen, Di- oder Polyaminoverbindungen, oder Aminoalkohole oder Mischungen daraus, mit einem linearen, verzweigten oder cyclischen, gesättigten, ungesättigten oder aromatischen Kohlenwasserstoffgerüst, ggf. oxo- oder azasubstituiert mit O- oder N-Atomen in der Kette, oder deren Umsetzungsprodukten mit Maleinsäureanhydrid, hergestellt werden oder nach der Herstellung mit diesen modifiziert werden.

7. Verfahren zur Herstellung der Copolymeren gemäß einem der Ansprüche 1 bis 6, dadurch gekennzeichnet, daß man die Amide oder die Ester und Amide α,β-ungesättigter Dicarbonsäuren oder deren Ammoniumsalze, insbesondere Maleinsäurederivate der allgemeinen Formeln (V), (VI) und (VII) 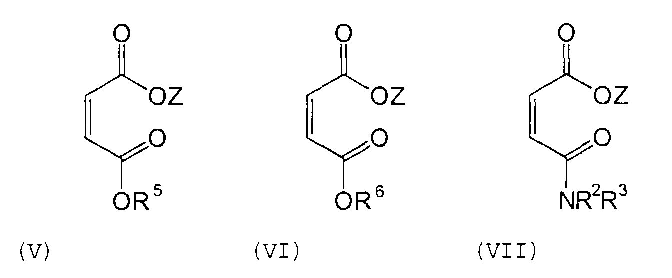 alleine oder im Gemisch miteinander, mit Ammoniak umsetzt und in das Polymer überführt, wobei Z für Wasserstoff oder Ammonium, R², R³, R⁵ und R⁶ für die oben genannten Reste stehen, gegebenenfalls in Gegenwart von bis zu 20 Gew.-%, bezogen auf copolymere Polyasparaginsäurederivate, proteinogener oder nicht proteinogener Aminosäuren oder deren Derivate, sowie gegebenenfalls in weiteren Schritten durch Hydrolyse Gruppen der Struktur der Formel (I), wobei R¹ die Bedeutung von R⁴, mit der oben genannten Definition von R⁴, hat, erzeugt.

8. Kosmetische W/O- oder O/W-Emulsionen, enthaltend copolymere Polyasparaginsäurederivate gemäß den Ansprüchen 1 bis 7.

9. Kosmetische Emulsionen nach Anspruch 8, dadurch gekennzeichnet, daß der nichtwäßrige Anteil 5 bis 99 Gew.-% an Ölkörpern aus der Gruppe der Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₂₀-Fettsäuren mit verzweigten Alkoholen, Ester von linearen und/oder verzweigten C₆-C₂₀-Carbonsäuren mit mehrwertigen Alkoholen und/oder Guerbetalkoholen, Triglyceride auf Basis von C₆-C₁₀-Fettsäuren, pflanzliche und tierische Öle und Fette, verzweigte primäre Alkohole, substituierte Cyclohexane, Guerbetcarbonate, Dialkylether und/oder aliphatische bzw. naphthenische Kohlenwasserstoffe enthält.

10. Kosmetische O/W-Emulsionen nach Anspruch 8 oder 9, dadurch gekennzeichnet, daß hydrophile Wachse aus der Gruppe der C₁₂-C₃₀-Fettalkohole, Wollwachsalkohole, C₁₆-C₂₂-Fettsäuren, Glycerinmono- und -diester und Sorbitanmono- und diester von gesättigten Fettsäuren mit 12 bis 22 C-Atomen enthalten sind.

11. Kosmetische Emulsionen nach einem der Ansprüche 8 bis 10, dadurch gekennzeichnet, daß ein oder mehrere Coemulgatoren aus der Gruppe der Anlagerungsprodukte von Ethylenoxid oder Ethylenoxid und Propylenoxid an C₁₂-C₃₀-Fettalkohole und Wollwachsalkohole, der Ethylenoxidanlagerungsprodukte von Glycerinmono- und -diestern und Sorbitanmono- und diestern von gesättigten und ungesättigten Fettsäuren mit 6 bis 22 C-Atomen, der Anlagerungsprodukte von Ethylenoxid und /oder Propylenoxid an Fettsäuren mit 12 bis 22 C-Atomen und an Alkylphenole mit 8 bis 15 C-Atomen in der Alkylgruppe, der C₁₂-C₁₈-Fettsäuremono- und -diester von Anlagerungsprodukten von Ethylenoxid an Glycerin, der Anlagerungsprodukte von Ethylenoxid an Fette und Öle, der Polyolester von gesättigten oder ungesättigten C₁₂-₂₂-Fettsäuren, auch verzweigte oder hydroxysubstituierte, Polysiloxan-Polyalkyl-Polyether-Copolymere und deren Derivate, der anionischen Tenside, kationischen Tenside, nichtionischen Tenside sowie zwitterionischen oder amphoteren Tenside enthalten sind.

12. Verwendung der Emulsionen nach einem der Ansprüche 8 bis 11 als Hautpflegemittel, Tagescreme, Nachtcreme, Pflegecreme, Nährcreme, Bodylotion, pharmazeutische Salbe und Lotion, Aftershavelotion und Sonnenschutzmittel.

13. Tensidische Zubereitungen für Reinigungsmittel und/oder kosmetische Mittel, enthaltend Polyasparaginsäurederivate nach einem der Ansprüche 1 bis 7, gegebenenfalls enthaltend ein oder mehrere weitere Tenside aus der Gruppe der anionischen, kationischen, nichtionischen, amphoteren und zwitter-ionischen Tenside sowie deren Mischungen daraus neben üblichen Hilfs- und Zusatzstoffen.

14. Verwendung der Tensidzubereitungen nach Anspruch 13 für Shampoos, Waschlotionen und Reinigungsmittel für Gesicht, Haar, Haut und Intimbereich, Rasiercreme und -lotionen, Flüssigseife, Geschirrspülmittel, Reinigungsmittel für glatte Oberflächen, Seifenstücke, Schaumbad, Duschgel sowie in Zahncreme und/oder Mundspülung.

15. Verwendung der Copolymeren gemäß einem der Ansprüche 1 bis 7 als Waschmittelhilfsstoff, Komplexagens für mehrwertige Kationen, Dispergierhilfsmittel für Lacke und Farben, Inkrustationsintibitor, Absorbermaterialien, Metalldesaktivatoren in Kunststoffen, als Hilfsstoffe in der Papier-, Leder und Textilindustrie oder als wirkungsverstärkende Zusatzstoffe zu Pestiziden oder Insektiziden.
